# EUROPEAN PATENT SPECIFICATION

(11) **EP 3 375 586 B1**
(45) Date of publication and mention of the grant of the patent: **16.06.2021**
(21) Application number: 16867891.0
(22) Date of filing: 10.11.2016
(51) Int. Cl.: B29C 41/40, A41D 19/04, A41D 19/015, A41D 19/00, B29C 41/14, B29C 33/56, B29K 105/00, B29L 31/48, B29L 31/00

(54) **FORMER FOR MANUFACTURING WALL THICKNESS-VARIABLE GLOVE AND GLOVE MANUFACTURED USING THE FORMER**
FORM ZUR HERSTELLUNG EINES WANDDICKENVARIABLEN HANDSCHUHS UND UNTER VERWENDUNG DER FORM HERGESTELLTER HANDSCHUH
FORME POUR FABRIQUER UN GANT À ÉPAISSEUR DE PAROI VARIABLE ET GANT FABRIQUÉ À L'AIDE DE LADITE FORME

(30) Priority: 25.11.2015 CN 201510831861
(43) Date of publication of application: 19.09.2018
(73) Proprietor: Institute of Materials, China Academy of Engineering Physics, Mianyang, Sichuan 621908 (CN)
(72) Inventor: ZHANG, Pengcheng, Mianyang Sichuan 621908 (CN); XU, Duigong, Mianyang Sichuan 621908 (CN); LIAO, Yichuan, Mianyang Sichuan 621908 (CN)
(74) Representative: Ipside
(86) International application number: PCT/CN2016/105261
(87) International publication number: WO 2017/088667

(56) References cited:
- WO-A1-2015/109546
- CN-A- 101 966 738
- CN-A- 105 479 643
- CN-U- 204 132 485
- CN-U- 205 143 586
- FR-A- 1 359 189
- JP-A- S4 987 455
- US-A- 3 859 410
- US-A- 4 135 867
- US-A1- 2003 124 354

## Description

### Field according to the present invention

The present invention relates to the field of protective equipment, in particular to the field of radiation protection products, and in particular to a former for manufacturing a wall thickness-variable glove and a glove manufactured using the former. The former may be used for manufacturing a wall thickness-variable protective glove, especially for manufacturing a wall thickness-variable X-ray protective glove. The manufactured glove may be used for protecting the hand back of a doctor in an interventional operation or orthopedic operation, and has better protective effects and application prospects.

### Description of the Related Art

Radiation protection rubber gloves used in the nuclear industry and nuclear medicine are iso-wall thickness gloves, which are generally manufactured by integral dipping using general glove formers. In order to achieve a better protective effect, gloves that are manufactured by integral dipping using general glove formers are generally thick and may affect the hand feeling of doctors in operations.

Chinese patent CN97225793.4 discloses an easy release film glove former. The glove former has a palm portion with a rough surface, and the rough surface is composed of evenly distributed arc-shaped concave parts, which allows easy release of the film glove.

Chinese patent CN200620124744.5 discloses a dipping stainless steel glove former. The glove former is of integral structure. An arm support is arranged at an arm portion of the former. The former is characterized by simple manufacture.

Chinese patent CN201410578601.0 discloses a former for manufacturing a TPE glove. The former comprises a palm former and an arm former. A concave ring groove is formed on the arm former, and a sleeve portion of the glove manufactured using the former is closed up. The glove manufactured using the former may be easily tightened on an arm of a user.

Patent document US4135867A describes a form on which elastomeric material may be deposited for making gloves, said form having a hand-forming portion, a cuff-forming portion and a plurality of finger-forming portions, all of said finger-forming portions being oriented in substantially the same plane as said hand-forming portion, said finger-forming and hand-forming portions having a recess of uniform thickness formed in both sides thereof which extends into each of said finger-forming portions and which covers the entire surfaces of said hand-forming and finger-forming portions except for a peripheral edge which surrounds said recess.

Patent document JPS4987455A1 discloses the applying of post-coating substance, however not a hydrophilic coating for local thickening.

Patent document US3859410A discloses a method for manufacturing gloves with the walls of the fingers and palm portions of a surgical glove which are as thin as possible consistent with adequate strength, and the wall of the cuff portion is relatively thick for strength in putting on and for gripping the wearer's gown, to preserve sensitivity for the wearer.

Patent document US 2003/124354 A1 discloses a method of making a colored, multi-layered polymer coated article.

Patent document FR 1 359 189A describes a glove which is provided to the shape of the hand, with the fingers having an initial curvature and the thumb in spaced position facing the palm of the hand, the natural position of the thumb by recalling at rest.

### Summary according to the present invention

The applicant has found through research that X-ray causes the most serious radiation damage to the hand backs of surgeons in the clinical application of X-ray machines. However, the existing iso-wall thickness lead protective gloves have poor flexibility in use and cannot meet the needs of doctors for flexible operations. Therefore, doctors are often reluctant to wear the existing iso-wall thickness lead protective gloves. As a result, there is an urgent need to develop a protective glove that has good X-ray protective effects and may meet the requirements of doctors for flexible operations, i. e. , a wall thickness-variable glove. Therefore, the utility model provides a former for manufacturing a wall thickness-variable glove.

With regard to the current problem that wall thickness-variable gloves cannot be manufactured as the existing iso-wall thickness X-ray protective gloves are manufactured by dipping using general formers, the purpose according to the present invention is to provide a former for manufacturing a wall thickness-variable glove and a glove manufactured using the former. The use of the former according to the present invention may achieve local thickening of the glove, thereby guaranteeing the flexibility of the glove on the premise of effective protection. In addition, the wall thickness-variable glove manufactured using the former according to the present invention may achieve local thickening of a hand back portion of the glove, so that the requirement for protection of the hand back of a doctor under an environment such as an interventional operation is met, especially the requirement for protection against X-ray may be met. The present invention has the advantages of reasonable design and convenient use, and the wall thickness-variable glove manufactured by one-time dipping has good application prospects.

In order to achieve the purpose, the present invention adopts the following technical solution:
A former for manufacturing a wall thickness-variable glove, characterized by comprising a base, an arm portion, a palm portion and finger portions, wherein the base, the arm portion, the palm portion and the finger portions are connected in sequence, the palm portion comprises a palm center member and a palm back member connected to the palm center member, the finger portions comprise a thumb member, a forefinger member, a middle finger member, a ring finger member and a little finger member, and a first recessed area with a depth of 0.3-1.5mm is formed on the palm back member;
the thumb member is provided with a second recessed area at one side of the palm back member, or the forefinger member is provided with a second recessed area at one side of the palm back member, or the thumb member, the forefinger member, the middle finger member, the ring finger member and the little finger member are provided with a second recessed area at one side of the palm back member respectively;
the second recessed area with a depth of 0.3-1.5mm is connected with the first recessed area;
and surfaces of the first recessed area and the second recessed area are respectively provided with a hydrophilic coating with a thickness of 1-300µm.

The palm center member and the palm back member are integrally formed.

The arm portion and the palm portion are hollow structures.

The arm portion, the palm portion and the finger portions are made of a ceramic material or a metal material respectively.

The area of the second recessed area on the thumb member is 0.1-0.7 time that of the thumb member at one side of the palm back member.

The area of the second recessed area on the forefinger member is 0.1-0.7 time that of the forefinger member at one side of the palm back member.

The area of the second recessed area on the thumb member, the forefinger member, the middle finger member, the ring finger member and the little finger member is 0.1-0.7 time that of the thumb member, the forefinger member, the middle finger member, the ring finger member and the little finger member at one side of the palm back member respectively.

The hydrophilic coating has a contact angle of 2-30°.

A glove manufactured using the former for manufacturing a wall thickness-variable glove.

With regard to the problem, the present invention provides a former for manufacturing a wall thickness-variable glove and a glove manufactured using the former. The former comprises a base, an arm portion, a palm portion and finger portions. The base, the arm portion, the palm portion and the finger portions are connected in sequence. The palm portion comprises a palm center member and a palm back member connected to the palm center member, the finger portions comprise a thumb member, a forefinger member, a middle finger member, a ring finger member and a little finger member, and a first recessed area with a depth of 0.3-1.5mm is formed on the palm back member. The former may be adopted for effectively thickening a hand back portion of the manufactured glove, and therefore the flexibility of other portions of the glove is guaranteed on the premise of effectively protecting the hand back of a doctor, and the characteristics of protection and use flexibility are both achieved, thus the former has good application prospects. The former provided by the present invention may be used for manufacturing a wall thickness-variable glove, so as to achieve local thickening of a hand back portion of the glove, and meet the requirement for protection of the hand back of a doctor under an environment such as an interventional operation.

Further, the thumb member is provided with a second recessed area at one side of the palm back member, or the forefinger member is provided with a second recessed area at one side of the palm back member; and the second recessed area with a depth of 0.3-1.5mm is connected with the first recessed area. Or the thumb member, the forefinger member, the middle finger member, the ring finger member and the little finger member are provided with a second recessed area at one side of the palm back member respectively, and the second recessed area with a depth of 0.3-1.5mm is connected with the first recessed area. The area of the second recessed area on the thumb member, the forefinger member, the middle finger member, the ring finger member and the little finger member is 0.1-0.7 time that of the thumb member, the forefinger member, the middle finger member, the ring finger member and the little finger member at one side of the palm back member respectively. With the structure, a rear half finger back and the palm back member of the former are depressed, thinner than a front half finger back of the fingers, and gradually depressed with a slope to form a 0.3-1.5mm deep shallow recessed area (i.e., the first recessed area and the second recessed area) capable of holding a latex. The hand back and the rear half fingers connected to the hand back of the glove manufactured using the former are locally thickened, and other portions are thin, therefore the hand back and the rear half fingers are effectively protected while the flexibility of the glove is guaranteed, thus the glove has good protective effects.

Further, surfaces of the first recessed area and the second recessed area are respectively provided with a hydrophilic coating with a thickness of 1-300µm. The hydrophilic coating has a contact angle of 2-30°.

The hydrophilicity or wettability of a solid surface is determined by chemical composition and micromorphology of the surface, and is generally measured by the size of the contact angle. The contact angle refers to the angle formed at the three-phase boundary by liquid droplets dripping on a solid surface. In general, a surface with a contact angle of <90° is defined as a hydrophilic surface, which appears as a surface that may be wetted with water; on the contrary, a surface with a contact angle of >90° is defined as a hydrophobic surface, the surface cannot be wetted with water; when the contact angle of a surface is close to 0°, the surface is called a superhydrophilic surface.

Surfaces of the first recessed area and the second recessed area according to the present invention are respectively provided with a hydrophilic coating, thereby forming a porous water-absorbing surface. Due to the arrangement of the hydrophilic coating, the contact angle of the surfaces of the first recessed area and the second recessed area is 2-30°, while the other surfaces of the former are not provided with a hydrophilic coating, so that the contact angle of other surfaces than the shallow recessed area (i.e., the first recessed area and the second recessed area) of the former is 30-70°. Based on the above structure, the hydrophilicity or wettability of the surfaces of the first recessed area and the second recessed area is superior to the surface of other portions, thus the structure is more hydrophilic and capable of carrying more coagulant and latex.

When the glove is manufactured using the former according to the present invention, one-time dipping forming may be achieved. Brief steps for manufacturing a wall thickness-variable glove using the former are described as follows: (1) the former is cleaned and dried, then dipped with a coagulant and dried; (2) the former treated in step (1) is dipped with a rubber latex or emulsion, then set and dried to obtain an initial blank; (3) the former treated in step (2) is vulcanized, released and dried to obtain a finished glove product of which the hand back portion is thickened and other portions are thinner.

The former according to the present invention may be used for manufacturing a wall thickness-variable protective glove, especially for manufacturing a wall thickness-variable x-ray protective glove. The manufactured glove may be used for protecting the hand back of a doctor in an interventional operation or orthopedic operation, and has better protective effects and application prospects.

Further, the arm portion and the palm portion are hollow structures, and the palm center member and the palm back member are integrally formed. The hollow structures help reduce the mass of the former.

The arm portion, the palm portion and the finger portions are made of a ceramic material or a metal material respectively. The former may be made of a ceramic material by isostatic pressing, conventional casting, investment casting, sintering, or hot injection molding; or the former may be made of a metal material by electroforming, conventional casting, machining, investment casting or rapid prototyping.

Based on the above structure, the mechanism for realizing local thickening of the glove according to the present invention is mainly based on the following three aspects: first, the first recessed area and the second recessed area may hold more latex, thus the thickening is achieved; second, the surfaces of the first recessed area and the second recessed area are more hydrophilic and may carry more latex; third, the surfaces of the first recessed area and the second recessed area are more hydrophilic and may carry more coagulant, and the coagulant has good water absorbability and may carry more latex after latex dipping. With the three aspects, the former according to the present invention may better achieve local thickening of the glove, and may be used for manufacturing a wall thickness-variable glove with good protective effects and flexible operation.

Further, the present invention provides a glove manufactured using the former for manufacturing a wall thickness-variable glove. The thickness of the thickened portion of the glove may be 0.3-2.5mm, and the thickness of unthickened portions may be 0.1-0.3mm.

In conclusion, a wall thickness-variable glove may be manufactured using the former according to the present invention, and the manufactured protective glove has the characteristics of good protective effects and flexible operation, and may particularly satisfy the requirement of protection against X-ray. The former according to the present invention may achieve local protection through the design of the first recessed area and the second recessed area, and finger pulp portions are thinner so as to ensure the flexibility of operation. The glove manufactured using the former according to the present invention is particularly suitable for interventional operations and orthopedic operations, the flexibility of the glove may be guaranteed on the premise of effective protection, thereby providing the general medical workers with practical and effective protection. The thickness of the thickened portion of the glove manufactured in the present invention may be 0.3-2.5mm, and the thickness of unthickened portions may be 0.1-0.3mm.

### Description of the Drawings

The present invention will be described in combination with examples and accompanied drawings, in which:

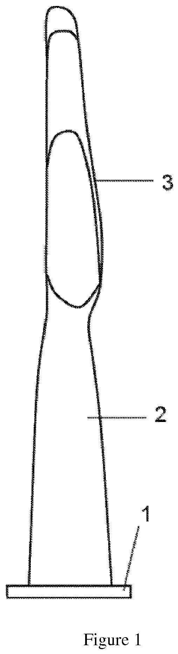

Figure 1 is a side view of example 1;
Figure 2 is a front view of Figure 1;
Figure 3 is a sectional view of Figure 2 in A-A direction;.

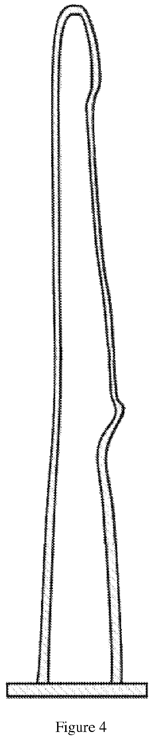

Figure 4 is a sectional view of Figure 2 in B-B direction;

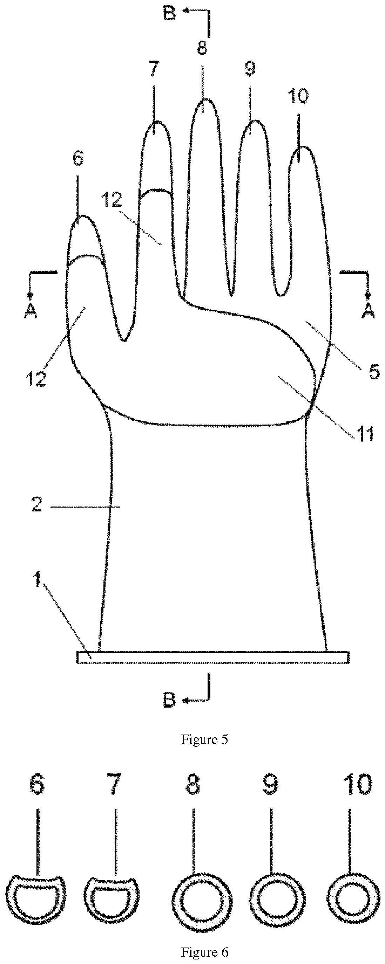

Figure 5 is a front view of example 2;
Figure 6 is a sectional view of Figure 5 in A-A direction; and

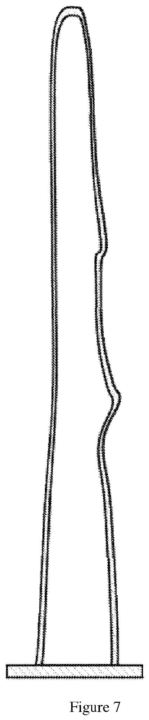

Figure 7 is a sectional view of Figure 5 in B-B direction.

Marks in the figures: 1-base, 2-arm portion, 3-palm portion, 4-palm center member, 5-palm back member, 6-thumb member, 7-forefinger member, 8-middle finger member, 9-ring finger member, 10-little finger member, 11-first recessed area, 12-second recessed area.

### Description of the Preferred Embodiment

All features or steps in all methods and procedures disclosed in the specification may be combined in any way, except mutually exclusive features and/or steps.

Any feature disclosed in the specification may be replaced with other equivalent or similar features, unless otherwise specified, that is, each feature is only an example of series of equivalent or similar features, unless otherwise specified.

### Example 1

As shown in the figures, the former comprises a base, an arm portion, a palm portion and finger portions. The base, the arm portion, the palm portion and the finger portions are connected in sequence as a whole. The arm portion and the palm portion are hollow structures. The finger portions comprise a thumb member, a forefinger member, a middle finger member, a ring finger member and a little finger member.

A first recessed area is formed on a palm back member. The thumb member, the forefinger member, the middle finger member, the ring finger member and the little finger member are provided with a second recessed area at one side of the palm back member respectively, and the second recessed area is connected with the first recessed area. The depth of the first recessed area and the second recessed area is 0.3-1.5mm respectively. The area of the second recessed area on the thumb member, the forefinger member, the middle finger member, the ring finger member and the little finger member is 0.5 time that of the thumb member, the forefinger member, the middle finger member, the ring finger member and the little finger member at one side of the palm back member respectively. Surfaces of the first recessed area and the second recessed area are respectively provided with a hydrophilic coating with a thickness of 1-300µm.

In the example, the former is made of a Al₂O₃ ceramic material by investment casting, and the contact angle of the surface of the former is 30-70°. The hydrophilic coating is prepared from a TiO₂ ceramic material by plasma spraying. The hydrophilic coating forms a porous water-absorbing surface with a contact angle of 2-30°.

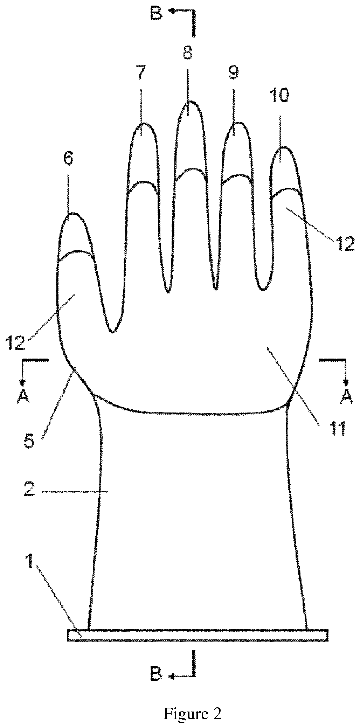

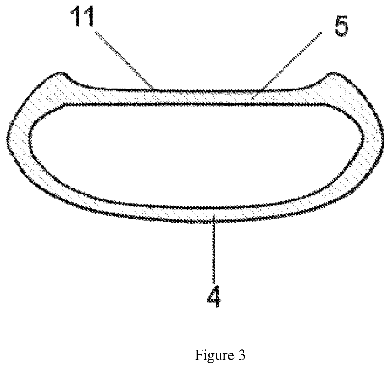

In the example, the thumb member, the forefinger member, the middle finger member, the ring finger member and the little finger member are provided with a second recessed area at one side of the palm back member respectively, that is, the front half finger back of the finger portions has a normal thickness, and the rear half finger back connected to the hand back is depressed, thus achieving thickening of the finger portions of the glove. Figure 3 is a sectional view of Figure 2 in A-A direction. As may be seen from Figure 3, a first recessed area is formed on the palm back member, and the palm back portion of the glove may be thickened by the first recessed area. Further, the base is provided with a connecting hole, the connecting hole is a through hole or a threaded hole which helps fix the base to the base.

Further, the steps for manufacturing the glove using the former of the example are as follows.
(1) The former is cleaned and dried, then dipped in a coagulant and dried, the coagulant is an aqueous CaCl₂ solution with a mass fraction of 3-20%.
(2) The former treated in step (1) is dipped with an emulsion, then set and dried. Typically but unrestrictedly, the dipping emulsion is mainly composed of a natural rubber latex, a compounding agent and a slurry containing a radiation protection material Bi₂O₃, and the compounding agent is mainly composed of a vulcanizing agent, a vulcanization accelerator, an antioxidant, a reinforcing agent and soft water.
(3) The former treated in step (2) is vulcanized, released and dried to obtain a finished glove product.

The thickness of the thickened portion of the glove manufactured in the example is 0.3-2.5mm, and the thickness of unthickened portions is 0.1-0.3mm.

### Example 2

As shown in the figures, the former comprises a base, an arm portion, a palm portion and finger portions. The base, the arm portion, the palm portion and the finger portions are connected in sequence as a whole. The arm portion and the palm portion are hollow structures. The finger portions comprise a thumb member, a forefinger member, a middle finger member, a ring finger member and a little finger member.

In the example, a first recessed area is formed on a palm back member. The thumb member and the forefinger member are provided with a second recessed area at one side of the palm back member respectively, and the second recessed area is connected with the first recessed area. The depth of the first recessed area and the second recessed area is 0.3-1.5mm respectively. Different from example 1, in the example, only the thumb member and the forefinger member are provided with a second recessed area respectively. The area of the second recessed area on the thumb member and the forefinger member is 0.5 time that of the thumb member and the forefinger member at one side of the palm back member respectively. Surfaces of the first recessed area and the second recessed area are respectively provided with a hydrophilic coating with a thickness of 100-200µm.

In the example, the former is made of stainless steel by investment casting, and the contact angle of the surface of the former is 30-70°. The hydrophilic coating is prepared from stainless steel by plasma spraying. The hydrophilic coating forms a porous water-absorbing surface with a contact angle of 2-30°.

Further, the steps for manufacturing the glove using the former of the example are as follows.
(1) The former is cleaned and dried, then dipped in a coagulant and dried, the coagulant is an aqueous CaCl₂ solution with a mass fraction of 3-20%.
(2) The former treated in step (1) is dipped with an emulsion, then set and dried. Typically but unrestrictedly, the dipping emulsion is mainly composed of a carboxylic butadiene-acrylonitrite latex, a compounding agent and a slurry containing a radiation protection material WO₃, and the compounding agent is mainly composed of a vulcanizing agent, a vulcanization accelerator, a reinforcing agent and soft water.
(3) The former treated in step (2) is vulcanized, released and dried to obtain a finished glove product.

The thickness of the thickened portion of the glove manufactured in the example is 0.3-2.5mm, and the thickness of unthickened portions is 0.1-0.3mm.

With convenient, simple and quick operation, the former of the example may be used to manufacture a wall thickness-variable glove by one-time dipping. The manufactured wall thickness-variable glove may effectively protect doctors on the premise of ensuring flexible operation, and may particularly satisfy the requirement of protection against X-ray, and has good application prospects.

## Claims

1. A former for manufacturing a wall thickness-variable glove by dipping, comprising a base (1), an arm
portion (2), a palm portion (3) and finger portions, wherein the base (1), the arm portion (2), the palm portion (3) and the finger portions are connected in sequence, the palm portion (3) comprises a palm center member (4) and a palm back member (5) connected to the palm center member (4), the finger portions comprise a thumb member (6), a forefinger member (7), a middle finger member (8), a ring finger member (9) and a little finger member (10), **characterized in that** a first recessed area (11) with a depth of 0.3-1.5mm is formed on the palm back member (5);
the thumb member (6) is provided with a second recessed area (12) at one side of the palm back member (5), or the forefinger member (7) is provided with a second recessed area (12) at one side of the palm back member (5), or the thumb member (6), the forefinger member (7), the middle finger member (8), the ring finger member (9) and the little finger member (10) are provided with a second recessed area (12) at one side of the palm back member (5) respectively;
the second recessed area (12) with a depth of 0.3-1.5mm is connected with the first recessed area (11); and surfaces of the first recessed area (11) and the second recessed area (12) are respectively provided with a hydrophilic coating with a thickness of 1-300µm.

2. The former for manufacturing a wall thickness-variable glove according to claim 1, **characterized in that** the palm center member (4) and the palm back member (5) are integrally formed.

3. The former for manufacturing a wall thickness-variable glove according to claim 1, **characterized in that** the arm portion (2) and the palm portion (3) are hollow structures.

4. The former for manufacturing a wall thickness-variable glove according to claim 1, **characterized in that** the arm portion (2), the palm portion (3) and the finger portions are made of a ceramic material or a metal material respectively.

5. The former for manufacturing a wall thickness-variable glove according to claim 1, **characterized in that** the area of the second recessed area (11) on the thumb member (6) is 0.1-0.7 time that of the thumb member (6) at one side of the palm back member (5).

6. The former for manufacturing a wall thickness-variable glove according to claim 1, **characterized in that** the area of the second recessed area (11) on the forefinger member (7) is 0.1-0.7 time that of the forefinger member (7) at one side of the palm back member (5).

7. The former for manufacturing a wall thickness-variable glove according to claim 1, **characterized in that** the area of the second recessed area (12) on the thumb member (6), the forefinger member (7), the middle finger member (8), the ring finger member (9) and the little finger member (10) is 0.1-0.7 time that of the thumb member (6), the forefinger member (7), the middle finger member (8), the ring finger member (9) and the little finger member (10) at one side of the palm back member (5) respectively.

8. The former for manufacturing a wall thickness-variable glove according to claim 1, **characterized in that** the hydrophilic coating has a contact angle of 2-30°.

9. A glove manufactured by dipping using the former for manufacturing a wall thickness-variable glove according to any of claims 1 to 8.

## Patentansprüche

1. Tauchform zum Herstellen eines wanddickenvariablen Handschuhs durch Tauchen, die eine Basis (1), einen Armabschnitt (2), einen Handflächenabschnitt (3) und Fingerabschnitte umfasst, wobei die Basis (1), der Armabschnitt (2), der Handflächenabschnitt (3) und die Fingerabschnitte der Reihe nach verbunden sind, der Handflächenabschnitt (3) ein Handinnenflächenelement (4) und ein Handrückenelement (5) umfasst, das mit dem Handinnenflächenelement (4) verbunden ist, die Fingerabschnitte ein Daumenelement (6), ein Zeigefingerelement (7), ein Mittelfingerelement (8), ein Ringfingerelement (9) und ein Element (10) für den kleinen Finger umfassen, **dadurch gekennzeichnet, dass** am Handrückenelement (5) ein erster vertiefter Bereich (11) mit einer Tiefe von 0,3-1,5 mm gebildet ist;
das Daumenelement (6) auf einer Seite des Handrückenelements (5) mit einem zweiten vertieften Bereich (12) versehen ist, oder das Zeigefingerelement (7) auf einer Seite des Handrückenelements (5) mit einem zweiten vertieften Bereich (12) versehen ist, oder das Daumenelement (6), das Zeigefingerelement (7), das Mittelfingerelement (8), das Ringfingerelement (9) und das Element (10) für den kleinen Finger auf einer Seite des Handrückenelements (5) jeweils mit einem zweiten vertieften Bereich (12) versehen sind;
der zweite vertiefte Bereich (12) mit einer Tiefe von 0,3-1,5 mm mit dem ersten vertieften Bereich (11) verbunden ist; und Oberflächen des ersten vertieften Bereichs (11) und des zweiten vertieften Bereichs (12) jeweils mit einer hydrophilen Beschichtung mit einer Dicke von 1-300 µm versehen sind.

2. Tauchform zum Herstellen eines wanddickenvariablen Handschuhs nach Anspruch 1, **dadurch gekennzeichnet, dass** das Handinnenflächenelement (4) und das Handrückenelement (5) einstückig gebildet sind.

3. Tauchform zum Herstellen eines wanddickenvariablen Handschuhs nach Anspruch 1, **dadurch gekennzeichnet, dass** es sich beim Armabschnitt (2) und dem Handflächenabschnitt (3) um Hohlstrukturen handelt.

4. Tauchform zum Herstellen eines wanddickenvariablen Handschuhs nach Anspruch 1, **dadurch gekennzeichnet, dass** der Armabschnitt (2), der Handflächenabschnitt (3) und die Fingerabschnitte aus einem Keramikmaterial bzw. einem Metallmaterial bestehen.

5. Tauchform zum Herstellen eines wanddickenvariablen Handschuhs nach Anspruch 1, **dadurch gekennzeichnet, dass** der Flächeninhalt des zweiten vertieften Bereichs (11) am Daumenelement (6) das 0,1-0,7-Fache desjenigen des Daumenelements (6) auf einer Seite des Handrückenelements (5) beträgt.

6. Tauchform zum Herstellen eines wanddickenvariablen Handschuhs nach Anspruch 1, **dadurch gekennzeichnet, dass** der Flächeninhalt des zweiten vertieften Bereichs (11) am Zeigefingerelement (7) das 0,1-0,7-Fache desjenigen des Zeigefingerelements (7) auf einer Seite des Handrückenelements (5) beträgt.

7. Tauchform zum Herstellen eines wanddickenvariablen Handschuhs nach Anspruch 1, **dadurch gekennzeichnet, dass** der Flächeninhalt des zweiten vertieften Bereichs (12) am Daumenelement (6), dem Zeigefingerelement (7), dem Mittelfingerelement (8), dem Ringfingerelement (9) und dem Element (10) für den kleinen Finger jeweils das 0,1-0,7-Fache desjenigen des Daumenelements (6), des Zeigefingerelements (7), des Mittelfingerelements (8), des Ringfingerelements (9) und des Elements (10) für den kleinen Finger auf einer Seite des Handrückenelements (5) beträgt.

8. Tauchform zum Herstellen eines wanddickenvariablen Handschuhs nach Anspruch 1, **dadurch gekennzeichnet, dass** die hydrophile Beschichtung einen Kontaktwinkel von 2-30° aufweist.

9. Handschuh, der durch Tauchen unter Verwendung der Tauchform zum Herstellen eines wanddickenvariablen Handschuhs nach einem der Ansprüche 1 bis 8 hergestellt wird.

## Revendications

1. Forme pour fabriquer un gant à épaisseur de paroi variable par trempage, comprenant une base (1), une portion bras (2), une portion paume (3) et des portions doigts, dans laquelle la base (1), la portion bras (2), la portion paume (3) et les portions doigts sont reliées de manière séquentielle, la portion paume (3) comprend un élément central de paume (4) et un élément arrière de paume (5) relié à l'élément central de paume (4), les portions doigts comprennent un élément pouce (6), un élément index (7), un élément majeur (8), un élément annulaire (9) et un élément auriculaire (10), **caractérisée en ce qu'**une première zone évidée (11) avec une profondeur de 0,3 à 1,5 mm est formée sur l'élément arrière de paume (5) ;
l'élément pouce (6) est pourvu d'une seconde zone évidée (12) au niveau d'un côté de l'élément arrière de paume (5), ou l'élément index (7) est pourvu d'une seconde zone évidée (12) au niveau d'un côté de l'élément arrière de paume (5), ou l'élément pouce (6), l'élément index (7), l'élément majeur (8), l'élément annulaire (9) et l'élément auriculaire (10) sont pourvus d'une seconde zone évidée (12) au niveau d'un côté de l'élément arrière de paume (5) respectivement ;
la seconde zone évidée (12) avec une profondeur de 0,3 à 1,5 mm est reliée à la première zone évidée (11) ; et des surfaces de la première zone évidée (11) et de la seconde zone évidée (12) sont pourvues respectivement d'un revêtement hydrophile avec une épaisseur de 1 à 300 µm.

2. Forme pour fabriquer un gant à épaisseur de paroi variable selon la revendication 1, **caractérisée en ce que** l'élément central de paume (4) et l'élément arrière de paume (5) sont formés d'un seul bloc.

3. Forme pour fabriquer un gant à épaisseur de paroi variable selon la revendication 1, **caractérisée en ce que** la portion bras (2) et la portion paume (3) sont des structures creuses.

4. Forme pour fabriquer un gant à épaisseur de paroi variable selon la revendication 1, **caractérisée en ce que** la portion bras (2), la portion paume (3) et les portions doigts sont faites d'un matériau céramique ou d'un matériau métallique respectivement.

5. Forme pour fabriquer un gant à épaisseur de paroi variable selon la revendication 1, **caractérisée en ce que** la superficie de la seconde zone évidée (11) sur l'élément pouce (6) est de 0,1 à 0,7 fois celle de l'élément pouce (6) au niveau d'un côté de l'élément arrière de paume (5).

6. Forme pour fabriquer un gant à épaisseur de paroi variable selon la revendication 1, **caractérisée en ce que** la superficie de la seconde zone évidée (11) sur l'élément index (7) est de 0,1 à 0,7 fois celle de l'élément index (7) au niveau d'un côté de l'élément arrière de paume (5).

7. Forme pour fabriquer un gant à épaisseur de paroi variable selon la revendication 1, **caractérisée en ce que** la superficie de la seconde zone évidée (12) sur l'élément pouce (6), l'élément index (7), l'élément majeur (8), l'élément annulaire (9) et l'élément auriculaire (10) est de 0,1 à 0,7 fois celle de l'élément pouce (6), l'élément index (7), l'élément majeur (8), l'élément annulaire (9) et l'élément auriculaire (10) au niveau d'un côté de l'élément arrière de paume (5) respectivement.

8. Forme pour fabriquer un gant à épaisseur de paroi variable selon la revendication 1, **caractérisée en ce que** le revêtement hydrophile a un angle de contact de 2 à 30°.

9. Gant fabriqué par trempage à l'aide de la forme pour fabriquer un gant à épaisseur de paroi variable selon l'une quelconque des revendications 1 à 8.
